# EUROPEAN PATENT APPLICATION

(11) **EP 2 138 165 A1**
(43) Date of publication of application: **30.12.2009**
(21) Application number: 08159289.1
(22) Date of filing: 27.06.2008
(51) Int. Cl.: A61K 9/20, A61K 9/28

(54) **Pharmaceutical composition comprising a statin**

(71) Applicant: KRKA, tovarna zdravil, d.d., Novo mesto, 8501 Novo mesto (SI)
(72) Inventor: Ocepek, Uros, 8000 Novo mesto (SI); Vrecer, Franc, 8351 Straza pri Novem mestu (SI)
(74) Representative: UEXKÜLL & STOLBERG

(57) **Abstract**

The invention relates to a solid pharmaceutical composition comprising at least one active agent selected from the group consisting of statins and the pharmaceutically acceptable salts thereof and at least one pharmaceutically acceptable low moisture excipient.

## Description

The invention relates to a pharmaceutical composition comprising a statin such as rosuvastatin, or a pharmaceutically acceptable salt thereof, as active ingredient.

### Background of the invention

The class of compounds referred to as "statins" is known to be inhibitors of 3-hydroxy-3-methylglutaryl-coenzyme A (HMG-CoA) reductase. Examples of compounds belonging to this class include atorvastatin, fluvastatin, lovastatin, cerivastatin, pravastatin, simvastatin and rosuvastatin. Due to their effectiveness as HMG-CoA reductase inhibitors, statins and their pharmaceutically acceptable salts are used in the treatment of *inter alia* hypercholesterolemia, hyperlipoproteinemia and atherosclerosis.

One particular statin is rosuvastatin, the chemical name of which is (E)-7-{4-(4-fluorophenyl)-6-isopropyl-2-[methyl(methylsulfonyl)amino]-pyrimidin-5-yl}-(3R,5S)-3,5-dihydroxy-hept-6-enoic acid:

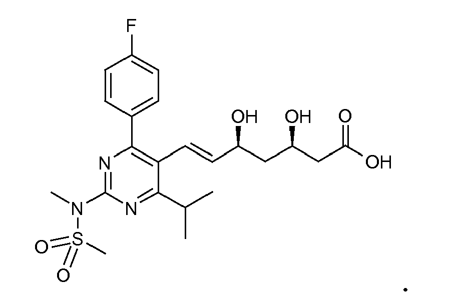

Rosuvastatin as well as its sodium and calcium salt are disclosed in EP 0 521 471 A1.

It has been found that statins are often difficult to formulate into stable pharmaceutical compositions, especially if the statin is used in amorphous form because of their susceptibility to degradation.

GB 2 262 229 A discloses a pharmaceutical composition comprising a HMG-CoA reductase inhibitor compound in combination with an alkaline medium capable of imparting a pH of at least 8 to an aqueous solution or dispersion of the composition. Preferably the alkaline medium is provided by sodium carbonate, sodium bicarbonate and/or calcium carbonate. However, such alkaline compounds have been found to have an irritating effect on the gastric mucosa.

According to WO 01/54668 A1 and WO 01/54669 A1 amorphous rosuvastatin calcium is stabilized by addition of an inorganic salt, particularly a tribasic phosphate salt in which the cation is multivalent, such as aluminum magnesium metasilicate, tribasic calcium phosphate, tribasic magnesium phosphate and tribasic aluminum phosphate. These stabilizers have also been found to have an irritating effect on the gastric mucosa.

WO 2008/035128 A1 teaches stabilization of rosuvastatin calcium by addition of magnesium hydroxide and/or calcium acetate or calcium gluconate or calcium glycerophosphate or aluminium hydroxide. WO 2008/062476 A1 discloses a pharmaceutical composition comprising at least one HMG-CoA reductase inhibitor in combination with an inorganic salt of monovalent cation.

WO 01/62230 A1 describes a process for the preparation of a granulate by granulating an active agent-containing solution or suspension with mannitol. When the active agent is a statin the granulate is typically formulated with NaOH.

WO 2007/071357 A2 discloses a pharmaceutical composition comprising rosuvastatin together with at least one additional ingredient selected from corn starch, mannitol, hydroxypropyl cellulose, silicified microcrystalline cellulose, croscarmellose sodium and hypromellose.

There remains a need for a pharmaceutical composition of a statin that is stable under pharmaceutical storage conditions and can be easily prepared without the need to work under special handling conditions, such as the use of organic solvents in the manufacture of the pharmaceutical composition, and wherein intestine irritating materials are substantially avoided.

### Description of the invention

It is an object of the present invention to provide a pharmaceutical composition comprising a statin, which composition is stable and can advantageously be prepared on an industrial scale.

It has surprisingly been found that this object can be achieved by a solid pharmaceutical composition comprising
(a) at least one active agent selected from the group consisting of statins and the pharmaceutically acceptable salts thereof, and
(b) at least one pharmaceutically acceptable low moisture excipient having a moisture content of less than 3.0 w/w%.

Preferably, the active agent is selected from the group consisting of atorvastatin, fluvastatin, lovastatin, cerivastatin, pravastatin, simvastatin and rosuvastatin and mixtures thereof and the pharmaceutically acceptable salts thereof.

A particularly preferred active agent is rosuvastatin, in particular the calcium salt thereof.

Rosuvastatin calcium can generally be prepared by any known process such as the processes described in EP 0 521 471 A1, WO 00/49014, WO 2007/099561 and WO2004/108691. A particularly advantageous process is disclosed in a co-pending application filed on June 25, 2008 ("Process for the preparation of rosuvastatin"; agent's file: P 77493).

It is further preferred that the active agent is present in amorphous form. Amorphous forms of statins and methods for their preparation are generally known in the art. For example, amorphous rosuvastatin calcium can be prepared according to any of the processes described in WO 2005/068435, WO 2005/040134, WO 2006/035277 and WO 2006/136407.

The amorphous active agent may further be milled. During milling the mechanical force exerted on the particle surface leads to particle size reduction. Milling can be performed by any milling process known in the art, for example using a ball mill (planetary ball mill or mixer mill), hammer mill, bead mill, disc mill, ultrasonic mill, torus mill, impact mill, vibration mill, pin mill or air jet mill.

The basic principle of treatment in an air jet mill is collision and attrition between particles suspended within the high velocity air stream which introduces the power to the milling chamber. In a ball mill, particles are fractured by impact of grinding media (e.g. balls, cubes, cylinders, jars etc.) that can occupy up to half of the mill chamber volume. Due to rotation of the chamber the grinding media falls from an elevated position. Friction is also present among all elements, contributing significantly to the attrition and consequently to the amorphous nature of the material being milled. One of the most widely used mills in the pharmaceutical industry is the hammer mill. In such equipment, particles are exposed to the impact of rapidly rotating hammers. During milling, material may additionally hit the perforated screen that is placed over the chamber outlet.

According to the present invention, the active agent preferably has an average particle size of less than 300 µm, preferably less than 250 µm.

The term "average particle size" as used herein refers to the volume mean diameter of particles. The diameter and volume mean diameter can be determined by laser light scattering using e.g. a Malvern-Mastersizer Apparatus MS 2000. Particle sizes are determined by measuring the angular distribution of laser light scattered by a homogeneous suspension of particles. The size distribution is determined from the light scattering data using the theory of light scattering developed by Gustav Mie.

According to a preferred embodiment of the invention, the active agent is only moderately hygroscopic. It is particularly preferred that the active agent has a low moisture binding capacity. As used herein, the term "low moisture binding capacity" means that the active agent binds not more than 15 w/w%, more preferably not more than 10 w/w%, still more preferably not more than 8 w/w% and most preferably not more than 5 w/w% of moisture at 25 °C and 90 % relative humidity. The moisture binding capacity may be determined by dynamic vapor sorption (DVS). Typical conditions for measuring moisture binding capacity using DVS include 2 cycles, 0 % - 90 % RH, 25 °C, step dm/dt = 0.002 %/min.

As used herein, the term "low moisture excipient" refers to an excipient having a moisture content of less than 3.0 w/w%. For example, the moisture content may be determined as loss on drying by halogen dryer (Mettler, 85°C, 20 minutes). Surprisingly, a stable solid pharmaceutical composition comprising a statin such as a rosuvastatin or atorvastatin salt can be produced by using low moisture excipient. It is further preferred that the at least one pharmaceutically acceptable low moisture excipient has a moisture content of less than 2.5 w/w%, particularly less than 2.0 w/w%, most preferably less than 1.5 w/w%.

According to a preferred embodiment of the invention, the low moisture excipient is capable of binding at least 6 w/w%, preferably at least 9 w/w% of moisture at 25 °C and 90 % relative humidity. The moisture binding capacity may be determined by dynamic vapor sorption (DVS) as described above.

According to a particularly preferred embodiment of the invention, the solid pharmaceutical composition comprises at least 20 w/w%, particularly at least 30 w/w%, most preferably at least 40 w/w% of the at least one pharmaceutically acceptable low moisture excipient.

Examples of excipients suitable for use in the present invention include diluents such as microcrystalline cellulose, lactose (e.g. α-lactose anhydrous or monohydrate, β-lactose), mannitol or starch or its derivatives such as pregelatinized starch, disintegrants such as croscarmellose sodium, polacryline potassium, low substituted hydroxypropyl cellulose or crospovidone, glidants such as colloidal silicium dioxide and/or talc, and lubricants such as fatty acid metal salts (e.g. magnesium stearate, calcium stearate, aluminium stearate, zinc stearate and/or sodium stearyl fumarate). Preferably, the diluent(s) and/or filler(s) have an average particle size in the range of from 10 to 1000 µm, preferably from 50 to 500 µm and most preferably from 100 to 300 µm.

According to a preferred embodiment of the invention, the at least one pharmaceutically acceptable low moisture excipient comprises microcrystalline cellulose. According to a particularly preferred embodiment, the at least one pharmaceutically acceptable low moisture excipient is microcrystalline cellulose. Surprisingly, a solid pharmaceutical composition of a statin comprising low moisture microcrystalline cellulose as a diluent are particularly stable under pharmaceutical storage conditions.

The pharmaceutical composition may comprise at least one further excipient. Typically, the at least one further excipient is selected from the group consisting of diluents, disintegrants, glidants and lubricants. Examples of suitable further excipients are those described above.

The pharmaceutical composition may further comprise additional pharmaceutically acceptable ingredients, such as e.g. surface active substances. However, it has been found that certain excipients, even though they may generally be pharmaceutically acceptable, have an irritating effect on the human gastrointestinal tract. It is therefore preferred that such ingredients are present in the composition only in low amounts, such as less than 2 w/w% or less than 0.5 w/w% based on the total weight of the composition. It is even more preferred that the composition is substantially free of such ingredients.

According to a particularly preferred embodiment, the pharmaceutical composition comprises less than 1 w/w%, particularly less than 0.5 w/w% of alkaline stabilizers. Most preferably, the pharmaceutical composition is substantially free of alkaline stabilizers. As used herein, the term "alkaline stabilizers" generally refers to compounds capable of imparting a pH of at least 8 to an aqueous solution or dispersion of the composition, wherein the pH is determined by taking a unit dosage of the pharmaceutical composition containing e.g. 20 mg of active agent and dispersing or dissolving the composition in 10 to 200 ml, in particular 100 ml, of water. Examples of alkaline stabilizers include inorganic hydroxide, carbonate and bicarbonate salts, of e.g. monovalent or divalent inorganic cations such as sodium, potassium, calcium or magnesium.

According to another preferred embodiment, a solution or dispersion of a unit dosage of the pharmaceutical composition containing e.g. 20 mg of active agent in 10 to 200 ml, in particular 100 ml, of water has a pH of less than 8, particularly in the range of from 6 to less than 8.

The solid pharmaceutical composition of the present invention can optionally be film-coated with a coating soluble in water. The coating may be applied onto tablet cores by aqueous or organic solvent based film-coating as generally known in the art. According to a particular embodiment, the solid pharmaceutical composition of present invention is coated with a film-coating having a low permeability for gases such as water vapor and/or oxygen. Preferably, the film coating has a water vapor permeability of below 300 g/m²·d (determined according to DIN 53122), preferably below 200 g/m²·d. Typically, the thickness of the film coating is at least 5 µm, particularly at least 10 µm and most preferably at least 15 µm.

Film coatings characterized by low permeability for gases such as water vapor and/or oxygen may be based on polymers such as polyvinyl alcohol (e.g. Opadry AMB), low viscosity hypromellose types, sodium carboxymethyl cellulose, aminoalkyl methycrylate copolymers (e.g. Eudragit E PO or Eudragit E 12,5). Further excipients such as plasticizers, anti-tacking agents, pigments and colorants may optionally be incorporated into the coating.

Film coatings can be applied onto tablet cores as a dispersion of polymer and further pharmaceutically acceptable ingredients, such as anti-tacking agents, plasticizers, pigments and/or colorants in a solvent or mixture of solvents selected from water and/or organic solvents such as alcohols (methanol, ethanol, isopropanol or the like), ketones (acetone). Suitable methods for applying film coatings to tablet cores are generally known in the art, such as coating of tablet cores in perforated coating drums like Manesty, Acela cota, GS or Glatt coating drums.

The invention also relates to a packaged pharmaceutical composition comprising the solid pharmaceutical composition described above, which is present in a low gas permeable primary packaging.

The low gas permeable primary packaging may comprise materials such as aluminium or polychloro-3-fluoroethylene homopolymer/PVC laminate. Typically, the thickness of the packaging will be in the range of 10 to 40 µm for Al/Al blisters and 10 to 110 µm for Al-polychloro-3-fluoroethylene homopolymer/PVC laminate blisters. Optionally, the packaged pharmaceutical composition may further comprise a desiccant. The desiccant may be placed inside the packaging unit together with a dosage form such as a tablet and/or in the closure system and/or can be incorporated into the walls of the primary packaging unit. For example, the pharmaceutical composition can be packaged in containers made of glass or polymers, with or without desiccant.

The invention further relates to a process for the preparation of the solid pharmaceutical composition or the packaged pharmaceutical composition described above, which process comprises compressing the active agent with at least one excipient in the absence of a solvent. According to one embodiment, the process comprises directly compressing a homogenous mixture of the active agent, the low moisture excipient(s) and optionally further excipients. According to another embodiment, the process comprises pre-granulating a mixture of the active agent, the low moisture excipient(s) and optionally further excipients in the absence of solvent and compressing the pre-granulated mixture. Pre-granulation may be achieved by dry granulation methods generally known in the art such as slugging or roller compaction. The composition may further be provided with a film coating as described above.

According to a particular embodiment of the invention, the solid pharmaceutical composition can be obtained by
(i) melt granulating the active agent, a low melting binder, at least one low moisture diluent capable of binding at least 6 w/w% of moisture at 25 °C and 90 % relative humidity, and optionally further excipients such as disintegrants,
(ii) optionally admixing other excipients selected from diluents, disintegrants, glidants and lubricants,
(iii) compressing the granulate or mixture into a core, and
(iv) optionally film coating the obtained core.

It is preferred that the low melting binder has a melting point below 80°C, particularly below 70°C and most preferably below 60°C. Examples of low melting binders include complex glycerides like Gelucire, poloxameres, sugar esters, polyethylene glycols having an average molecular weight in the range of from 1.500 to 10.000, and the like.

The process according to the invention may further comprise drying steps. According to one embodiment, a mixture of active agent with at least one excipient can optionally be dried prior to being compressed or compacted. Drying may also be applied to tablet cores manufactured by direct compression or to granulate and/or tablets produced by dry granulation processes such as compaction on a roller compactor or slugging. Suitable drying methods are generally known in the art and include drying by passing dry air (preferably air comprising less than 3 g of water per 1 kg of air) or dry nitrogen over the mixture, granulate or tablet to be dried, drying at elevated temperature at normal or reduced air pressure or microwave drying. Preferably, drying is continued until the residual moisture (determined by loss on drying by halogen dryer as described above) in the mixture, granulate or tablet to be dried is less than 2.5 w/w%, preferably less than 2.0 w/w%.

According to a particular embodiment of the invention, the low moisture excipient can be prepared by drying an excipient having a normal moisture content, such as microcrystalline cellulose having a moisture content of 3 to 5 w/w%, with drying methods generally known in the art, such as any one of the drying methods described above, before incorporating it into the solid pharmaceutical composition.

The process for the preparation of the solid pharmaceutical composition or the packaged pharmaceutical composition can optionally be performed under conditions of reduced relative humidity of the surrounding atmosphere, e.g. at conditions, wherein the relative humidity of the surrounding atmosphere is below 40 %, preferably below 35 %, to prevent moisture sorption from the surrounding atmosphere into the composition.

The process can optionally be performed in the dark or under red light in order to minimize degradation of active substance by light exposure.

To avoid oxidative degradation of active agent and other ingredients susceptible to such degradation, the composition may be packaged into a primary packaging, such as a low gas permeable primary packaging, in an inert atmosphere such as nitrogen, argon or xenon. This will provide for a decreased concentration of oxygen in the atmosphere surrounding the dosage form in the primary packaging such as for example a blister, strip, glass or plastic container, such as a securitainer. As used herein, the term "decreased concentration of oxygen" means that the concentration of oxygen in the atmosphere surrounding the individual dosage form such as tablet or capsule is below 10 vol.-%, particularly below 7.5 vol.-%, more preferably below 5 vol.-% and most preferably below 2.5 vol.-%.

According to a particular aspect, the invention is directed to an active agent selected from the group consisting of statins and the pharmaceutically acceptable salts thereof having a low moisture binding capacity as defined above, i.e. wherein the active agent binds not more than 15 w/w%, more preferably not more than 10 w/w%, still more preferably not more than 8 w/w% and most preferably not more than 5 w/w% of moisture at 25 °C and 90 % relative humidity. Preferably, the active agent is selected from the group consisting of atorvastatin, fluvastatin, lovastatin, cerivastatin, pravastatin, simvastatin and rosuvastatin and mixtures thereof and the pharmaceutically acceptable salts thereof. Rosuvastatin, the calcium salt of rosuvastatin, and more specifically amorphous rosuvastatin calcium are particularly preferred. Moreover, it has surprisingly been found that active agents having a low moisture binding capacity can advantageously be prepared by reducing the particle size of the active agent to an average particle size of less than 300 µm, preferably less than 250 µm. Reduction of particle size may for example be performed using a milling method as described above. Active agents having a low moisture binding capacity have been found to be particularly useful for the preparation of solid pharmaceutical compositions.

The invention will be further illustrated by way of the following examples.

### Examples

### Preparation of amorphous rosuvastatin calcium

### Example 1

Generally following the process disclosed in EP 0 521 471, example 7, the sodium salt of rosuvastatin (101 g, 200 mmol) was dissolved in water (1011 ml) at room temperature under a nitrogen atmosphere. 1 M CaCl₂ (202 ml) was slowly added (1 ml/min), and the mixture was stirred at room temperature for 2 h. Precipitated product was collected by filtration, washed with water and vacuum dried (2 h, 50 °C). Powdered amorphous calcium salt of rosuvastatin was obtained (100 g, 100 %). The average particle size of the particles was determined to be 224 µm.

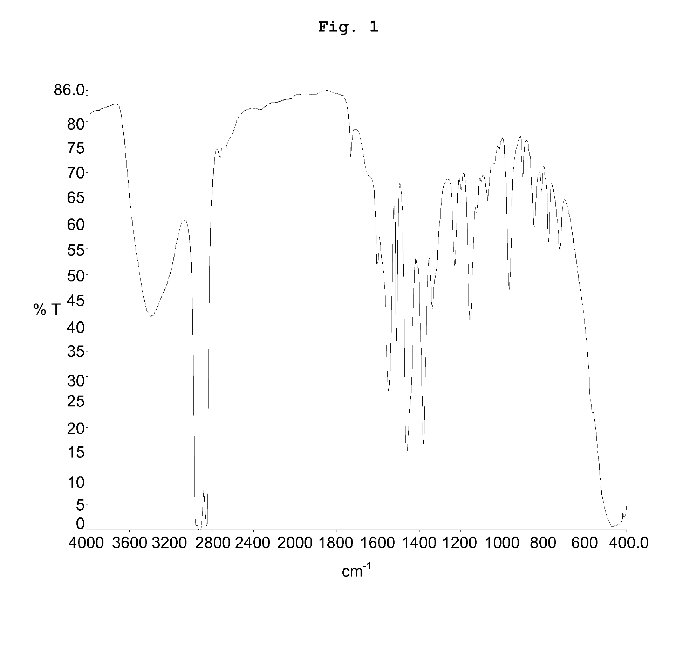

Fig. 1 shows an FT-IR spectrum of the amorphous rosuvastatin calcium obtained above. The spectrum was recorded on a FT-IR System SPECTRUM GX Perkin Elmer in paraffin oil [Range: 4000-400 cm⁻¹, Resolution: 4 cm⁻¹].

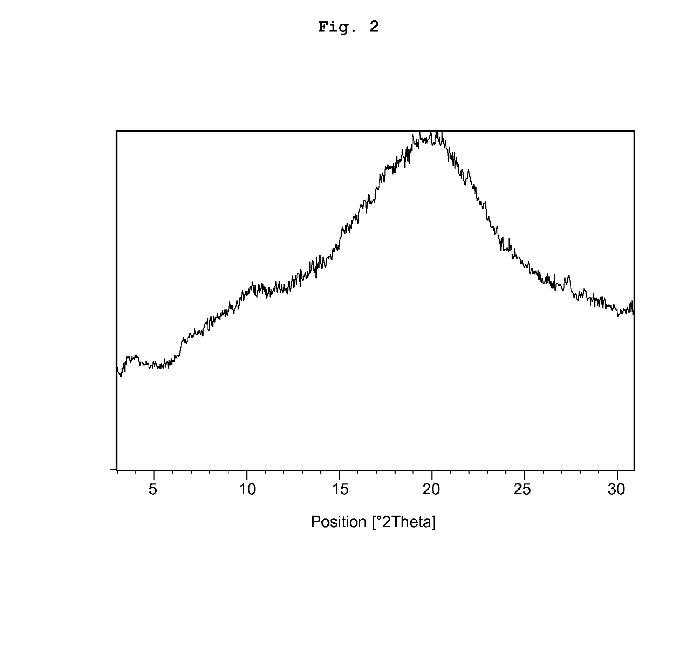

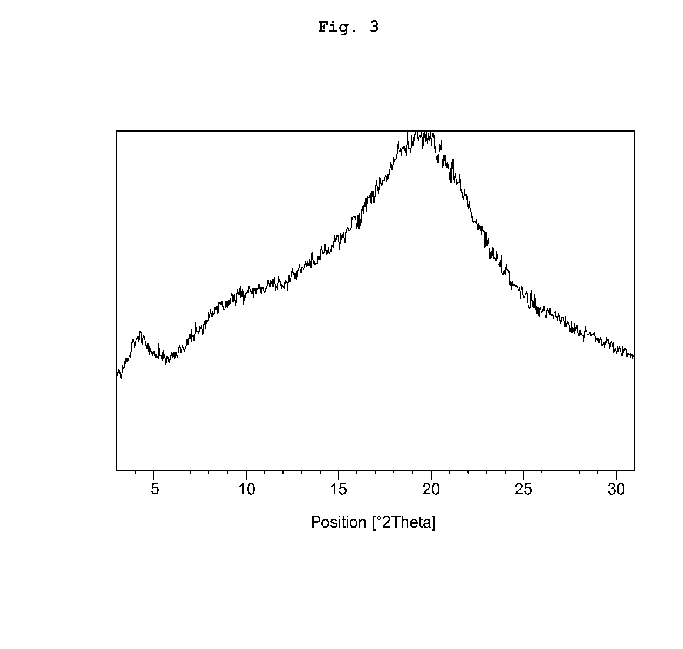

The obtained compound was subsequently milled in a Retsch Mixer Mill MM 200, operated at 20 Hz (1200 rpm) for 20 minutes. The XRD diffractogram of the milled compound (shown in Fig. 3) was identical to the XRD diffractogram of the compound before the milling (shown in Fig. 2). Both XRD diffractograms were recorded on a Phillips PW3040/60 X'Pert PRO diffractometer using CuKα radiation (1,541874 Å).

Hygroscopicity of the compound before and after milling was determined by dynamic vapour sorption (DVS) (2 cycles, 0% - 90% RH, T = 25°C, step dm/dt=0,002 (%/min)). Prior to milling, the amorphous rosuvastatin calcium obtained above had a moisture sorption of 34.23 % at 25 °C and 90 % RH. After ball milling, the moisture sorption of the amorphous rosuvastatin calcium was determined to be 4.77 % at 25 °C and 90% RH.

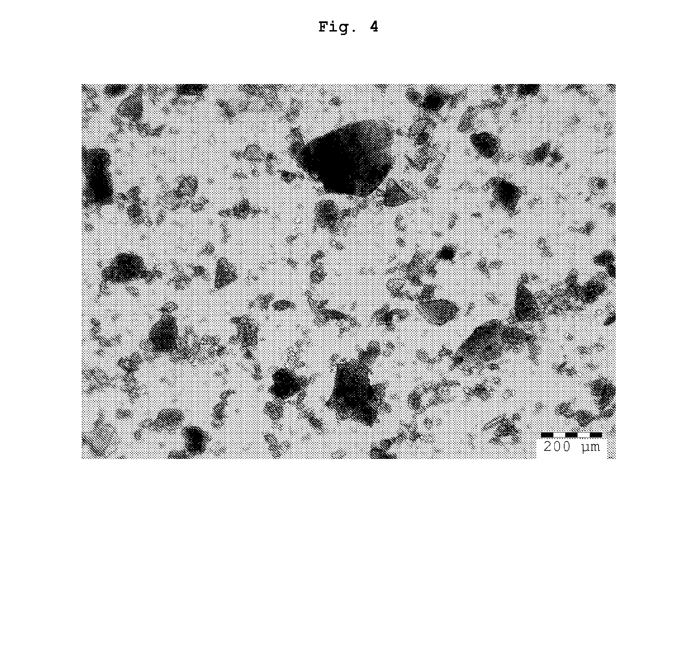

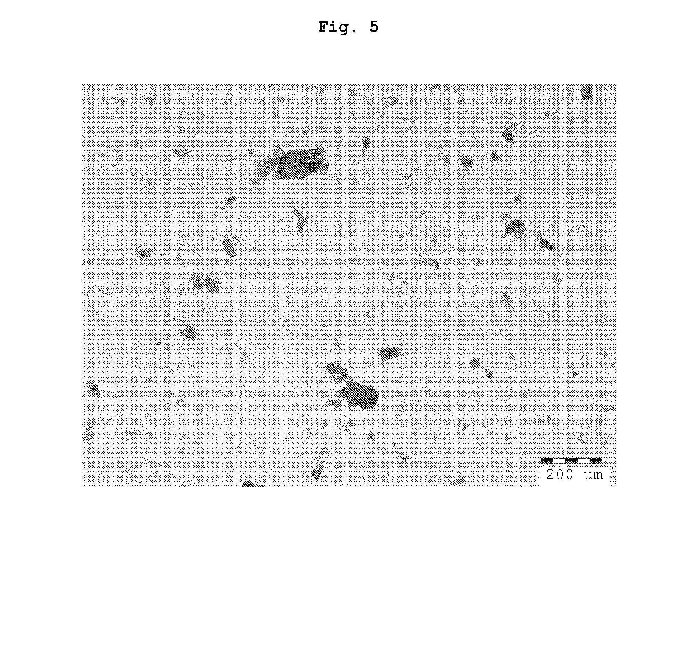

The particle size distribution of the amorphous rosuvastatin calcium obtained above was determined by a laser light scattering method using a Malvern Mastersizer 2000 Apparatus with Silcon fluid F10 as the dilution medium. 100-800 mg of substance were dispersed in 5 to 10 ml of non-polar dispersant and then subjected to the size determination. Fig. 4 and Fig. 5 show particles before and after milling, respectively.

### Pharmaceutical compositions of rosuvastatin calcium

### Example 2

Solid pharmaceutical compositions in form of tablets were prepared by direct compression of a homogenous mixture of the ingredients as shown in the table below.

| | **Amount [mg]** | **% weight in composition** |
|---|---|---|
| Rosuvastatin Ca amorphous | 41.67 | 13.89 |
| Pharmatose DCL-22 | 164.65 | 54.88 |
| Avicel PH-112 | 54.92 | 18.31 |
| Hydrotalcite | 20.00 | 6.67 |
| Crosscarmellose-Na | 15.00 | 5.00 |
| Mg stearate | 3.76 | 1.25 |

### Example 3

Solid pharmaceutical compositions in form of tablets were prepared by direct compression of a homogenous mixture of the ingredients as shown in the table below.

| | **Amount** | **% weight in** |
|---|---|---|
| | **[mg]** | **composition** |
| Rosuvastatin Ca amorphous | 41.67 | 13.89 |
| Pharmatose DCL-22 | 184.65 | 61.55 |
| Avicel PH-112 | 54.92 | 18.31 |
| Crosscarmellose-Na | 15.00 | 5.00 |
| Mg stearate | 3.76 | 1.25 |

### Example 4

Solid pharmaceutical compositions in form of tablets were prepared by direct compression of a homogenous mixture of the ingredients as shown in the table below.

| | **Amount** | **% weight in** |
|---|---|---|
| | **[mg]** | **composition** |
| Rosuvastatin Ca amorphous | 43.34 | 14.35 |
| Aerosil | 0.66 | 0.22 |
| Avicel PH-200LM | 140.00 | 46.36 |
| Pharmatose DCL-22 | 100.00 | 33.11 |
| Kollidon CL | 15.00 | 4.97 |
| Mg stearate | 3.00 | 0.99 |

### Example 5

The pharmaceutical compositions obtained according to Examples 2 to 4 were compared with a product currently marketed under the name Crestor^{®} (40 mg film coated tablets), which has a composition as disclosed in EP 1 223 918 B1.

Following an accelerated stability test scheme, uncoated compositions were stored at 80 °C and 80 °C/75 % RH for 4 days.

Both before (t = 0) and after exposition to accelerated stability testing conditions, the amount of total impurities present in the compositions was measured using HPLC (area-%). The difference in the amount of total impurities is given in the following table:

| | **Difference [%] compared to t = 0 of total impurities measured by HPLC** | |
|---|---|---|
| | **80 °C** | **80 °C/75 % RH** |
| | **4 days** | **4 days** |
| Example 2 | 0.7 | 1.11 |
| Example 4 | 0.63 | 2.53 |
| Crestor^{®} 40 mg | 3.14 | 2.61 |

### Example 6

Solid pharmaceutical compositions in form of tablets were prepared by direct compression of a homogenous mixture of the ingredients as shown in the table below.

| | **Amount** | | **% weight in** | **Amount** | | **% weight in** | **Amount** | **% weight in** |
|---|---|---|---|---|---|---|---|---|
| | **[mg]** | | **composition** | **[mg]** | | **composition** | **[mg]** | **composition** |
| | **80 mg** | **40 mg** | | **20 mg** | **10 mg** | | **5 mg** | |
| | **tablets** | **tablets** | | **tablets** | **tablets** | | **tablets** | |
| Rosuvastatin Ca amorphous | 83.16 | 41.58 | 13.86 | 20.79 | 10.40 | 6.93 | 5.20 | 3.47 |
| | | | | | | | | |
| Aerosil | 1.32 | 0.66 | 0.22 | 0.66 | 0.33 | 0.22 | 0.33 | 0.22 |
| Avicel PH-200LM | 329.52 | 164.76 | 54.92 | 175.55 | 87.77 | 58.51 | 90.47 | 60.31 |
| Pharmatose DCL-22 | 160 | 80 | 26.67 | 90 | 45 | 30.00 | 47.5 | 31.67 |
| | | | | | | | | |
| Kollidon CL | 20 | 10 | 3.33 | 10 | 5 | 3.33 | 5 | 3.33 |
| Mg stearate | 6 | 3 | 1.00 | 3 | 1.5 | 1.00 | 1.5 | 1.00 |
| Core weight [mg] | 600 | 300 | | 300 | 150.00 | | 150.00 | |
| Film coating | 18 | 9 | | 9 | 4.5 | | 4.5 | |
| Total weight [mg] | 618 | 309 | | 309 | 154.50 | | 154.50 | |

Constituents were blended in the following order:
- Amorphous rosuvastatin calcium and Aerosil,
- Avicel PH-200 LM,
- Pharmatose DCL-22 and Kollidon CL,
- Mg stearate.

The obtained homogenous blend was compressed into tablet cores on a tableting machine. The obtained cores were subsequently film coated to a coating weight corresponding to about 3 w/w% of the tablet core weight as described in Example 6A or Example 6B below.

### Example 6A

The cores obtained according to Example 6 were film coated with Opadry AMB (a ready-to-use water based film-coating suspension comprising 18 w/w% of solids).

### Example 6B

The cores obtained according to Example 6 were coated with a coating dispersion having the following composition:

| | | |
|---|---|---|
| Eudragit E 100 | 2.5 | w/w% |
| 30 w/w% pigment suspension | 7.5 | w/w%^{*} |
| Acetone | 7.0 | w/w% |
| Isopropanol | 81.5 | w/w% |
| Water | 1.5 | w/w% |

| | | |
|---|---|---|
| *Calculated on weight of dry substances | | |

**Composition of 30 w/w% pigment suspension:**

| | |
|---|---|
| Talc | 16% |
| Titanium dioxide | 4% |
| Macrogol 6000 | 4% |
| Red ferric oxide | 6% |
| Water | 6% |
| Isopropanol | 64% |

### Example 7A/B

Solid pharmaceutical compositions in form of tablets were prepared by compaction of active ingredient and microcrystalline cellulose (Avicel PH-112) and then mixing and compressing with the remaining ingredients.

| | **Amount [mg]** | | **% weight in composition** | **Amount [mg]** | | **% weight in composition** | **Amount [mg]** | **% weight in composition** |
|---|---|---|---|---|---|---|---|---|
| | **80 mg tablets** | **40 mg tablets** | | **20 mg tablets** | **10 mg tablets** | | **5 mg tablets** | |
| Rosuvastatin Ca amorphous | 83.16 | 41.58 | 13.86 | 20.79 | 10.40 | 6,93 | 5.20 | 3.47 |
| Aerosil | 1.32 | 0.66 | 0.22 | 0.66 | 0.33 | 0,22 | 0.33 | 0.22 |
| Avicel PH-112 | 329.52 | 164.76 | 54.92 | 175.55 | 87.77 | 58,51 | 90.47 | 60.31 |
| Pharmatose DCL-22 | 160 | 80 | 26.67 | 90 | 45 | 30,00 | 47.5 | 31.67 |
| Kollidon CL | 20 | 10 | 3.33 | 10 | 5 | 3,33 | 5 | 3.33 |
| Mg stearate | 6 | 3 | 1.00 | 3 | 1.5 | 1.00 | 1.5 | 1.00 |
| Core weight [mg] | 600 | 300 | | 300 | 150.00 | | 150.00 | |
| Film coating | 30 | 15 | | 15 | 4.5 | | 7.5 | |
| Total weight [mg] | 630 | 315 | | 315 | 154.50 | | 157.50 | |

The pharmaceutical composition was prepared in the following manner:
- Amorphous rosuvastatin calcium, Aerosil and Avicel PH-112 were blended, the blend was compacted on a roller compactor and subsequently milled to obtain a granulate;
- the obtained granulate was mixed with Pharmatose DCL-22 and Kollidon CL; and
- Mg stearate was admixed to the obtained mixture.

The obtained homogenous blend was compressed into tablet cores on a tableting machine. The obtained cores were subsequently film coated to a coating weight corresponding to about 5 w/w% of the tablet core weight analogously to the process described for Example 6A (Example 7A) or Example 6B (Example 7B).

### Example 8A/B

Solid pharmaceutical compositions in form of tablets were prepared by direct compression of a homogenous mixture of the ingredients as shown in the table below.

| | **Amount [mg]** | | **% weight in composition** | **Amount [mg]** | | **% weight in composition** | **Amount [mg]** | **% weight in composition** |
|---|---|---|---|---|---|---|---|---|
| | **80 mg tablets** | **40 mg tablets** | | **20 mg tablets** | **10 mg tablets** | | **5 mg tablets** | |
| Rosuvastatin Ca amorphous | 83.16 | 41.58 | 13.86 | 20.79 | 10.40 | 6.93 | 5.20 | 3.47 |
| Aerosil | 1.32 | 0.66 | 0.22 | 0.66 | 0.33 | 0.22 | 0.33 | 0.22 |
| Avicel PH-200 LM | 329.52 | 164.76 | 54.92 | 175.55 | 87.77 | 58.51 | 90.47 | 60.31 |
| Pharmatose DCL-22 | 150 | 75 | 25.00 | 85 | 42.5 | 28.33 | 45 | 30.00 |
| Hydrotalcite | 10 | 5 | 1.67 | 5 | 2.5 | 1.67 | 2.5 | 1.67 |
| Kollidon CL | 20 | 10 | 3.33 | 10 | 5 | 3.33 | 5 | 3.33 |
| Mg stearate | 6 | 3 | 1.00 | 3 | 1.5 | 1.00 | 1.5 | 1.00 |
| Core weight [mg] | 600 | 300 | | 300 | 150 | | 150.00 | |
| Film coating | 18 | 9 | | 9 | 4.5 | | 4.5 | |
| Total weight [mg] | 618 | 309 | | 309 | 154.5 | | 154.50 | |

Constituents were blended in following order:
- Amorphous rosuvastatin calcium, Hydrotalcite and Aerosil,
- Avicel PH-200 LM,
- Pharmatose DCL-22 and Kollidon CL,
- Mg stearate.
   The obtained homogenous blend was compressed into tablet cores on a tableting machine. The obtained cores were subsequently film coated to a coating weight corresponding to about 3 w/w% of the tablet core weight analogously to the process described for Example 6A (Example 8A) or Example 6B (Example 8B).

### Example 9A/B

Solid pharmaceutical compositions in form of tablets were prepared by compaction of active ingredient, microcrystalline cellulose (Avicel PH-112) and hydrotalcite and then mixing and compressing with the remaining ingredients.

| | **Amount [mg]** | | **% weight in composition** | **Amount [mg]** | | **% weight in composition** | **Amount [mg]** | **% weight in composition** |
|---|---|---|---|---|---|---|---|---|
| | **80 mg tablets** | **40 mg tablets** | | **20 mg tablets** | **10 mg tablets** | | **5 mg tablets** | |
| Rosuvastatin Ca amorphous | 83.16 6 | 41.58 | 13.86 | 20.79 | 10.40 | 6.93 | 5.20 | 3.47 |
| Aerosil | 1.32 | 0.66 | 0.22 | 0.66 | 0.33 | 0.22 | 0.33 | 0.22 |
| Avicel PH-200 LM | 329.52 | 164.76 | 54.92 | 175.55 | 87.77 | 58.51 | 90.47 | 60.31 |
| Pharmatose DCL-22 | 150 | 75 | 25.00 | 85 | 42.5 | 28.33 | 45 | 30.00 |
| Hydrotalcite | 10 | 5 | 1.67 | 5 | 2.5 | 1.67 | 2.5 | 1.67 |
| Kollidon CL | 20 | 10 | 3.33 | 10 | 5 | 3.33 | 5 | 3.33 |
| Mg stearate | 6 | 3 | 1.00 | 3 | 1.5 | 1.00 | 1.5 | 1.00 |
| Core weight [mg] | 600 | 300 | | 300 | 150 | | 150.00 | |
| Film coating | 18 | 9 | | 9 | 4.5 | | 4.5 | |
| Total weight [mg] | 618 | 309 | | 309 | 154.5 | | 154.50 | |

The pharmaceutical composition was prepared in following manner:
- Amorphous rosuvastatin calcium, Hydrotalcite, Aerosil and Avicel PH-112 were blended, the blend was compacted on a roller compactor and subsequently milled to obtain a granulate;
- the obtained granulate was mixed with Pharmatose DCL-22 and Kollidon CL; and
- Mg stearate was admixed to the obtained mixture.

The obtained homogenous blend was compressed into tablet cores on a tableting machine. The obtained cores were subsequently film coated to a coating weight corresponding to about 3 w/w% of the tablet core weight analogously to the process described for Example 6A (Example 9A) or Example 6B (Example 9B).

### Example 10A/B

Solid pharmaceutical compositions in form of tablets were prepared by direct compression of a homogenous mixture of the ingredients as shown in the table below.

| | **Amount [mg]** |
|---|---|
| Rosuvastatin Ca amorphous | 20.79 |
| Aerosil | 0.66 |
| Avicel PH-200 LM | 175.55 |
| Mannitol DC | 85 |
| Hydrotalcite | 5 |
| Kollidon CL | 10 |
| Mg stearate | 3 |
| Core weight [mg] | 300 |
| Film coating | 9 |
| Total weight [mg] | 309 |

Constituents were blended in following order:
- amorphous rosuvastatin calcium and hydrotalcite,
- Aerosil and Avicel PH-200 LM,
- mannitol DC and Kollidon CL,
- Mg stearate.

The obtained homogenous blend was compressed into tablet cores on a tableting machine. The obtained cores were subsequently film coated to a coating weight corresponding to about 3 w/w% of the tablet core weight analogously to the process described for Example 6A (Example 10A) or Example 6B (Example 10B).

### Example 11A/B

A solid pharmaceutical composition in form of tablets was prepared by direct compression of a homogenous mixture of the ingredients as shown in the table below.

| | **Amount [mg]** |
|---|---|
| Rosuvastain Ca amorphous | 20.79 |
| Aerosil | 0.66 |
| Avicel PH-200 LM | 155.55 |
| Mannitol DC | 85 |
| Hydrotalcite | 5 |
| L-HPC LH-11 | 30 |
| Mg stearate | 3 |
| Core weight [mg] | 300 |
| Film coating | 9 |
| Total weight [mg] | 309 |

Constituents were blended in following order:
- Amorphous rosuvastatin calcium and Hydrotalcite,
- Aerosil and Avicel PH-200 LM,
- Mannitol DC and L-HPC LH-11,
- Mg stearate.

The obtained homogenous blend was compressed into tablet cores on a tableting machine. The obtained cores were subsequently film coated to a coating weight corresponding to about 3 w/w% of the tablet core weight analogously to the process described for Example 6A (Example 11A) or Example 6B (Example 11B).

## Claims

1. A solid pharmaceutical composition comprising
(a) at least one active agent selected from the group consisting of statins and the pharmaceutically acceptable salts thereof, and
(b) at least one pharmaceutically acceptable low moisture excipient having a moisture content of less than 3.0 w/w%.

2. The pharmaceutical composition according to claim 1, wherein the active agent is selected from the group consisting of atorvastatin, fluvastatin, lovastatin, cerivastatin, pravastatin, simvastatin and rosuvastatin and mixtures thereof and the pharmaceutically acceptable salts thereof.

3. The pharmaceutical composition according to claim 2, wherein the active agent is amorphous rosuvastatin calcium.

4. The pharmaceutical composition according to any one of claims 1 to 3, wherein the active agent binds not more than 15 w/w%, more preferably not more than 10 w/w%, still more preferably not more than 8 w/w% and most preferably not more than 5 w/w% of moisture at 25 °C and 90 % relative humidity.

5. The pharmaceutical composition according to any one of claims 1 to 4, wherein the pharmaceutically acceptable low moisture excipient has a moisture content of less than 2.5 w/w%, particularly less than 2.0 w/w%, most preferably less than 1.5 w/w%.

6. The pharmaceutical composition according to any one of claims 1 to 5, wherein the pharmaceutically acceptable low moisture excipient is capable of binding at least 6 w/w%, preferably at least 9 w/w% of moisture at 25 °C and 90 % relative humidity.

7. The pharmaceutical composition according to any one of claims 1 to 6 comprising at least 20 w/w%, particularly at least 30 w/w%, most preferably at least 40 w/w% of the pharmaceutically acceptable low moisture excipient.

8. The pharmaceutical composition according to any one of claims 1 to 7, wherein the pharmaceutically acceptable low moisture excipient comprises microcrystalline cellulose.

9. The pharmaceutical composition according to any one of claims 1 to 8 further comprising at least one further excipient selected from the group consisting of diluents, disintegrants, glidants and lubricants.

10. The pharmaceutical composition according to any one of claims 1 to 9, which comprises less than 2 w/w%, particularly less than 0.5 w/w% of alkaline stabilizers, most preferably is substantially free of alkaline stabilizers.

11. The pharmaceutical composition according to any one of claims 1 to 10, wherein a solution or dispersion of the pharmaceutical composition has a pH of less than 8, particularly in the range of from 6 to less than 8.

12. A packaged pharmaceutical composition comprising the solid pharmaceutical composition according to any one of claims 1 to 11, wherein the solid pharmaceutical composition is present in a low gas permeable primary packaging.

13. A process for the preparation of the pharmaceutical composition according to any one of claims 1 to 11 or the packaged pharmaceutical composition according to claim 12, which process comprises compressing the active agent with at least one excipient in the absence of solvent and optionally coating the obtained composition and/or packaging the obtained composition.

14. The process according to claim 13 comprising directly compressing a homogenous mixture of the active agent, the low moisture excipient(s) and optionally further excipients.

15. The process according to claim 13 comprising pre-granulating a mixture of the active agent, the low moisture excipient(s) and optionally further excipients in the absence of solvent and compressing the pre-granulated mixture.

16. An active agent selected from the group consisting of statins and the pharmaceutically acceptable salts thereof, wherein the active agent binds not more than 15 w/w%, more preferably not more than 10 w/w%, still more preferably not more than 8 w/w% and most preferably not more than 5 w/w% of moisture at 25 °C and 90 % relative humidity.

17. The active agent according to claim 16, wherein the active agent is amorphous rosuvastatin calcium.

18. A process for preparing an active agent according to claim 16 or 17, comprising reducing the particle size of the active agent to an average particle size of less than 300 µm, preferably less than 250 µm.

19. Use of an active agent according to claim 16 or 17 for the preparation of a solid pharmaceutical composition.
